# EUROPEAN PATENT APPLICATION

(11) **EP 3 296 746 A1**
(43) Date of publication of application: **21.03.2018**
(21) Application number: 16306199.7
(22) Date of filing: 20.09.2016
(51) Int. Cl.: G01N 33/68, G01N 33/74

(54) **IN VITRO METHOD FOR DIAGNOSING AT EARLY STAGE INTESTINAL ISCHEMIA**

(71) Applicant: Université de Bourgogne, 21078 Dijon Cedex (FR); Institut National Supérieur des Sciences Agronomiques, de l'Alimentation et de l'Environnement, 21000 Dijon (FR); Institut National de la Santé et de la Recherche Médicale, 75013 Paris 13 (FR)
(72) Inventor: GROBER, Jacques, 21310 BELLENEUVE (FR); LEBRUN, Lorène, 21000 DIJON (FR)
(74) Representative: Pontet Allano & Associes

(57) **Abstract**

The present invention concerns an *in vitro* diagnostic method for diagnosing at an early stage an intestinal ischemia in a patient suspected of suffering from intestinal ischemia.

## Description

The present invention concerns an *in vitro* method for diagnosing at early stage of intestinal ischemia.

Acute mesenteric ischemia (AMI) is a severe medical emergency caused by a sudden decrease of blood flow through the mesenteric vessels, as a result of some diseases, trauma, shock, surgery, or organ transplantation. Mesenteric ischemia leads to cellular dysfunction and eventual gangrene of the bowel wall. AMI may be classified as either arterial or venous. Arterial AMI may be also classified as a nonocclusive mesenteric ischemia (NOMI) or an occlusive mesenteric arterial ischemia (OMAI). In large part because of the association with atherosclerosis, AMI is commonly considered a disease of the older population, with the typical age of onset being older than 60 years (Cardin et al., Aging Clin Exp Res. 2012 Jun. 24 (3 suppl): 43-46).

If mesenteric ischemia itself is deleterious for an organism, its treatment which consists in a reperfusion, could be worse. The bowel is one of the organs the most sensible to ischemia-reperfusion (Yamamoto et al., J. Surg. res., 2001, 99: 134-141). Intestinal mucosa can be rapidly impacted by a hypoperfusion (lack of oxygen). However, paradoxically, restoration of blood flow after ischemia (reperfusion) of ischemically damaged intestinal tissue may further aggravate tissue damage rather than decrease tissue damage as it abruptly reintroduces oxygen that stimulates the production of free radicals that promote or accelerate necrosis (Guan et al., Am J Physiol Gastrointest Liver Physiol 2009, 297: G187-G196). Ischemia followed by reperfusion (IR) can rapidly trigger the production of inflammation mediators. This local inflammation can be quickly generalized at the systemic level. The passing of pro-inflammatory bacterial endotoxins through intestinal barrier can result in systemic inflammation response syndrome (SIRS) state and dysfunction of other organs. Besides, intestinal ischemia-reperfusion is considered as a motor of multiple organ failure (MOF) state (Harvard et al., J. Vasc. Surg. 1993, 18: 459-469).

Despite the last medical advances, the high mortality of this pathology has not changed since the 1940s: it concerns about 60% to 80% of patients suffering from the said disease (Schoots et al., Br. J. Surg. 2004 Jan. 91(1): 17-27). Once bowel wall infarction has occurred, mortality may be as high as 90%. Unfortunately, even with a good treatment, there is still 50 to 80% of the patients that die.

This high mortality is partially due to the difficulty to diagnose AMI at early stage. Before the appearance of warning signs of peritonitis, symptoms of AMI are initially nonspecific. Until now, there is no efficient biological marker which enables to diagnose AMI at early stage. Conventional clinical used markers are serum lactate level and white blood cell number. These markers have neither satisfactory specificity, nor sensibility and cannot diagnose AMI at early stage. Other existing diagnostic tests are angiography or tomodensitometry. They present many disadvantages, since they are invasive and could generate medical complications, such as those at the renal level (Glenister et Corker, ANSZ J. Surg. 2004, 74: 260-265).

In the last few years, a great clinical and preclinical effort has been done for seeking for biomarkers which could predict gastro-intestinal damage before it is generated to systematic level. D-lactate (Demir et al., Dig. Surg., 2012, 29: 226-235), intestinal fatty acid binding protein "I-FABP" (Cronk et al., Curr. Surg. 2006, 63: 322-325), α-gLutathione S-transferase (Khurana et al., 2002, J. Pediatr. Surg., 2002, 37: 1543-1548), albumin (Dundar et al., Acad. Emerg. Med. 2010, 17: 1233-1238) and D-dimer (Chiu et al., Am. J. Emerg. Med. 2009, 27: 975-979) are studied as potential biomarker for AMI diagnostic. I-FABP is particularly promising, since the concentration of plasma I-FABP in mouse is increased 30 minutes post ischemia followed by 2 hours of reperfusion, which enables to predict intestinal ischemia prior to the appearance of pathological evidence (Khadaroo et al., PloS One, 2014, 9: e115242).

Despite these promising results, I-FABP can detect ischemia only 30 min post ischemia. As such, it remains necessary to develop new biomarkers that have the capacity to diagnose AMI more rapidly and at more early stages.

The Inventors have surprisingly found in a mouse model of intestinal ischemia/reperfusion, that when a mouse suffers from intestinal ischemia, an increase of circulating glucagon-like peptide (GLP-1) in the plasma level of the said mouse can be observed after only 10 minutes of ischemia followed by 15 minutes of reperfusion. In another word, GLP-1 is predictive at a very early stage of an intestinal ischemia in mice.

These experimental results suggest that GLP-1 could also be predictive at a very early stage of an intestinal ischemia in human or other non-human mammals, particularly in patient suspected of suffering from intestinal ischemia.

The circulating GLP-1 protein is mainly secreted by intestinal L-cells predominantly localized in the distal small intestine and colon. The circulating GLP-1 forms are: GLP-1-(7-37) and GLP-1-(7-36)NH₂.

Therefore, the first aspect of the invention relates to an *in vitro* diagnostic method for diagnosing at an early stage an intestinal ischemia in a patient suspected of suffering from intestinal ischemia, which comprises:
(i) determining in a biological sample from said patient the circulating glucagon-like peptide 1 (GLP-1) level,
(ii) comparing said level with the circulating glucagon-like peptide 1 level in a reference sample ,
wherein the increase of said level in the patient is indicative of said patient suffering from intestinal ischemia.

"A patient suspected of suffering from intestinal ischemia" refers to a patient who suffers from one of the diseases which are generally known as causes of intestinal ischemia or has predisposing conditions, such as a patient suffering from a cardiac emboli, emboli from fragments of proximal aortic thrombus, atheromatous plaque dislodged by arterial catheterization or surgery, an atherosclerotic vascular disease, aortic aneurysm, aortic dissection, arteritis, decreased cardiac output from myocardial infarction or congestive heart failure, dehydration from any cause, hypotension from congestive heart failure, myocardial infarction, sepsis, aortic insufficiency, severe liver or renal disease, or recent major cardiac or abdominal surgery, or a patient consuming vasopressors, ergotamines, cocaine, or digitalis, or a patient having hypercoagulability from protein C and S deficiency, antithrombin III deficiency, dysfibrinogenemia, abnormal plasminogen, polycythemia vera, thrombocytosis, sickle cell disease, factor V Leiden mutation, pregnancy, and oral contraceptive use, tumor causing venous compression or hypercoagulability, intra-abdominal infections, such as appendicitis, diverticulitis, or abscess, venous congestion from cirrhosis, venous trauma from accidents or surgery, especially portacaval surgery, increased intra-abdominal pressure from pneumoperitoneum during laparoscopic surgery, pancreatitis, decompression sickness.

The term "at early stage" refers to a period as short as 10 minutes of ischemia followed by 15 minutes of reperfusion.

The term "biological sample" refers to any biological sample which can be obtained from a patient, in particular a sample of body fluids, such as urine or blood. In a preferred embodiment of the present invention, the biological sample is a blood plasma sample.

A reference sample used in the *in vitro* method of the present invention is a biological sample obtained from a healthy subject.

According to the present invention, when GLP-1 level in a biological sample of a patient suspected of suffering from intestinal ischemia is at least 50%, 60%, 70%, 80%, 90%, 100%, 150%, 200%, 250%, or 300% higher than GLP-1 level measured at the same time in reference sample, it is considered that there is an increase of GLP-1 level in said patient and there is very high probability that said patient is suffering from an intestinal ischemia.

An "intestinal ischemia" is referred to a variety of disorders that occur when blood flow in the gastrointestinal tract is insufficient, which can affect small intestine (mesenteric ischemia) or colon (ischemic colitis). The method of the present invention can be used for *in vitro* diagnosis of mesenteric ischemia-reperfusion, especially acute mesenteric ischemia or chronic mesenteric ischemia, ischemic colitis, or a disease or trouble linked to gut barrier. In a preferred embodiment, the method of the present invention is for *in vitro* diagnosing acute mesenteric ischemia.

According to the method of the present invention, the level of circulating glucagon-like peptide 1 is determined by the mean value of an immunoassay.

An immunoassay is understood as different immunological techniques known from the one skilled in the art such as ELISA (Enzyme-Linked Immunosorbent Assay), Western-blot, RIA (radioimmunoassay), competitive EIA (competitive enzyme immunoassay), immunocytochemistry and immunohistochemistry techniques.

In a particular embodiment, the immunoassay is an ELISA test, which uses at least one antibody with specificity for GLP-1. Said antibody can be a commercially available monoclonal or polyclonal antibody directed against GLP-1.

The ELISA test can be carried out by a commercially available GLP-1 ELISA test.

Said ELISA assay can be direct ELISA, indirect ELISA, sandwich ELISA, or competitive ELISA.

In a direct ELISA test, the presence of GLP-1 is directly indicated by a specific antibody conjugated with an enzyme; while in an indirect ELISA test, GLP-1 is recognized by a first antibody specific to GLP-1 protein, said first antibody being recognized by a second antibody conjugated with an enzyme.

The sandwich ELISA quantifies GLP-1 protein between two layers of antibodies (i.e. capture and detection antibody). Either monoclonal or polyclonal antibodies can be used as the capture and detection antibodies in Sandwich ELISA systems.

Another aspect of the present invention concerns the use of a reagent capable of detecting the level of circulating glucagon-like peptide 1 for diagnosing *in vitro* at an early stage an intestinal ischemia in a patient suspected of suffering from intestinal ischemia.

In a preferred embodiment, said reagent is an antibody directed against glucagon-like peptide 1. Said antibody can be any conventional or commercially available monoclonal or polyclonal antibody known in prior art.

Particularly, the present invention concerns the use of a kit of ELISA containing an antibody directed against glucagon-like peptide 1 for diagnosing *in vitro* at an early stage an intestinal ischemia in a patient suspected of suffering from intestinal ischemia.

The ELISA test can be carried out by a commercially available GLP-1 ELISA test.

The present invention is explained more in detail by following figures and examples.

### Figures

Figures 1A, 1B and 1C show intestinal injury following acute mesenteric ischemia and reperfusion. Hematoxylin / eosin staining of ileum histological sections after sham treatment (figure 1A), 20 minutes of ischemia followed by 15 minutes of reperfusion (figure 1B) and 40 minutes of ischemia followed by 15 minutes of reperfusion (figure 1C).

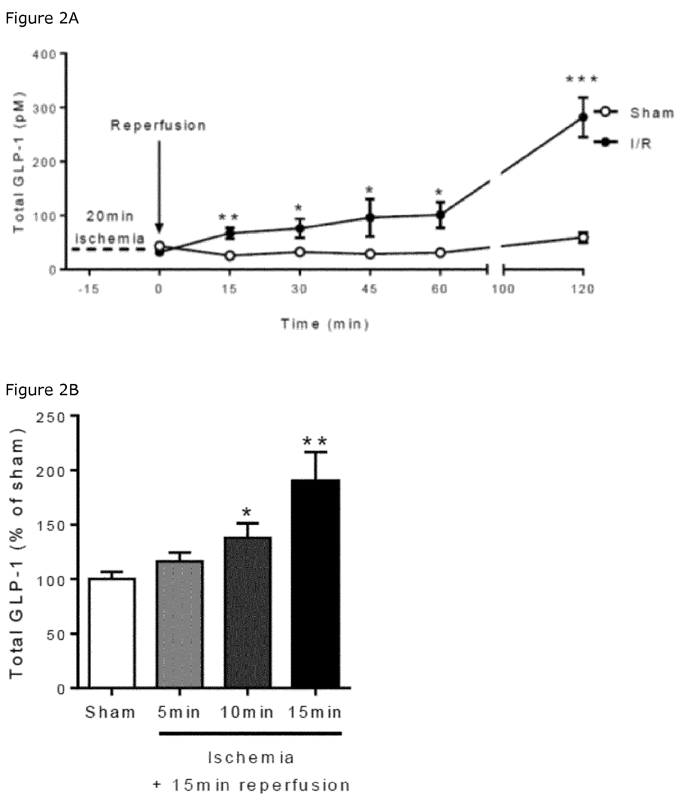

Figures 2A and 2B show that intestinal ischemia/reperfusion induces a quick secretion of GLP-1. Ischemia-reperfusion (I/R) of the superior mesenteric artery is applied in a group of 6 mice. Sham-operated mice were used as control. Statistical analysis were performed using an unpaired t test, difference from sham (*) are displayed: *p<0.05, **p<0.01 and ***p<0.001. Values are mean ± SEM. Figure 2A: total GLP-1 plasma levels (pM; n=5) after 20 minutes of ischemia followed by 15, 30, 45, 60 and 120 minutes of reperfusion. Figure 2B: total GLP-1 plasma levels (% of sham; n=5) after 5, 10 and 15 minutes of ischemia followed by 15min of reperfusion.
Figure 3: total GLP-1 and I-FABP plasma levels (% of sham, n=6) after a short and a long I/R (20 minutes ischemia followed by 15 minutes or 2 hours reperfusion respectively).
Figure 4 shows the comparison of the secretion of inflammation markers after a treatment of I/R (20min/30min) in mice of Sham group and of I/R group.

### Examples

### 1. Materials and methods

### Animals

WT mice (8-12 weeks old, Charles River) from a homogeneous C57BL6/J background were housed in a controlled environment and fed a standard chow diet (A03 diet; Safe, Augy, France). Animals had free access to water and food. All experiments involving animals were performed in accordance with institutional guidelines and approved by the University of Burgundy's Ethics Committee on the Use of Laboratory Animals (protocol number 5459).

### Animal model of intestinal ischemia/reperfusion

Mice were separated into sham-operated groups and ischemia/reperfusion (I/R) groups (n=5/6). They were anesthetized with isoflurane inhalation and placed in a supine position on heating pads to maintain body temperature at 37°C. Midline laparotomy was performed and the superior mesenteric artery (SMA) was isolated. Ischemia was induced by clamping the SMA for 5, 10, 15 or 20 minutes and was followed by 15, 30, 45, 60 or 120 minutes of reperfusion (removal of the clamp). Gut ischemia was confirmed by intestinal color, change and gut reperfusion by the reappearance of pulsation and color. Blood collections were performed to quantify GLP-1 and cytokines. Mice were euthanatized by cervical dislocation and the distal part of the small intestine (ileum) was removed and immediately fixed for histological studies.

Surgical operation for sham-operated group mice was the same, except that the superior mesenteric artery was not clamped.

### Light microscopy

The morphologic alterations in the gut were examined by light microscopy (x50, x100 and x200). Briefly, tissues from the distal small intestine (ileum) were promptly taken in sham-operated and I/R groups after 20 or 40 minutes of ischemia and 15 minutes of reperfusion. Gut samples were fixed for 48 hours in 10% neutral buffered formalin at room temperature, dehydrated by graded ethanol and embedded in paraffin for histological analysis. Tissue sections (thickness of 5µm) were deparaffinized with xylene, stained with hematoxylin and eosin.

### Blood and plasma collection

Blood samples were collected in EDTA-coated tubes (BD Vacutainer®) from the systemic (retro-orbital or intracardiac puncture) circulation. Plasma was separated by centrifugation at 8000 rpm for 10 minutes at 4°C. Blood and plasma samples were frozen at -20°C for further analysis.

### Biochemical Analysis

Total GLP-1 and I-FABP concentrations were determined by commercially available ELISA Kits (Millipore, St. Charles, MO and Cliniscience) in accordance with manufacturer's protocols.

Cytokines plasma levels (interleukin (IL)-1β, IL-6 and tumor necrosis factor-α (TNF-α)) were measured by Milliplex MAP 5-Plex Kit using mouse cytokine/chemokine magnetic bead panel (Millipore, Billerica, MA) according to the manufacturer's protocol and using a LuminexR apparatus (Bio-Plex 200, Bio-Rad).

### Statistical Analysis

Numeric data are presented as mean ± standard error of mean. Statistical analysis were performed using either the unpaired Sudent's t-test or the nonparametric Mann-Withney U test depending on data distribution's normality. D'Agostino's K² test was used to establish whether or not groups of data were normally distributed. A statistical correction was applied when variances were different between groups. A value of P < 0.05 was considered statistically significant.

### 2. Results

### Rapid GLP-1 secretion after gut barrier injury

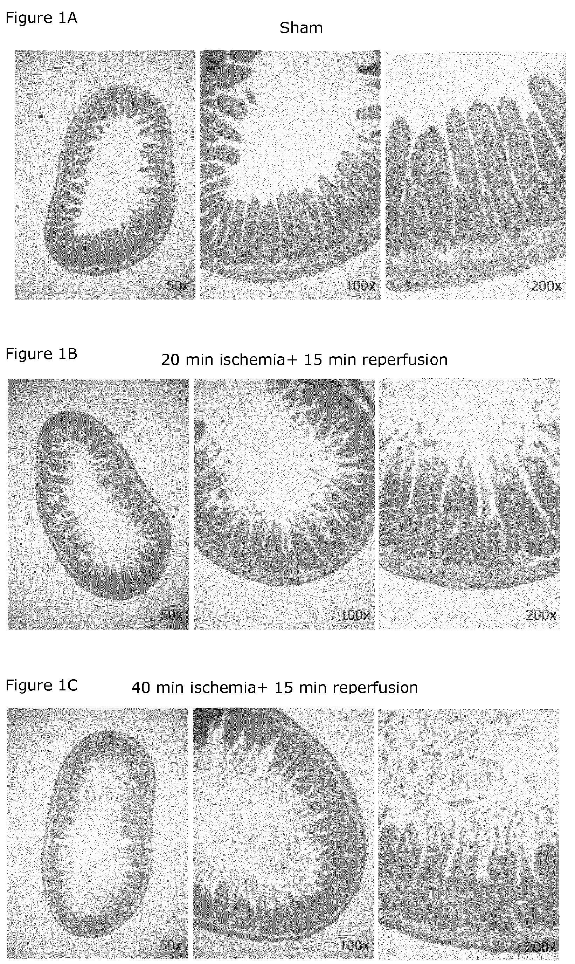

The mesenteric ischemia-reperfusion (I/R) was produced in a group of mice. A group of sham-operated mice was used as control. Gut ultrastructure was deeply disorganized after I/R (Figures 1A, 1B, 1C). Short times of I/R were sufficient to damage intestinal villi compared to sham-operated mice (Figure 1A and 1B). Increased damages were observed with increased times of I/R (Fig. 1C). I/R experiments led to a rapid increase of GLP-1 plasma levels (Figures 2A and 2B). As shown in Fig. 2A, reperfusion of the mesenteric artery for 2 hours after 20 minutes of ischemia led to a raise of GLP-1 plasma levels. More notably, this increase was significant after only 15 minutes of reperfusion. Shorter times of ischemia (< 20 minutes) were still able to induce GLP-1 secretion (Fig 2B).

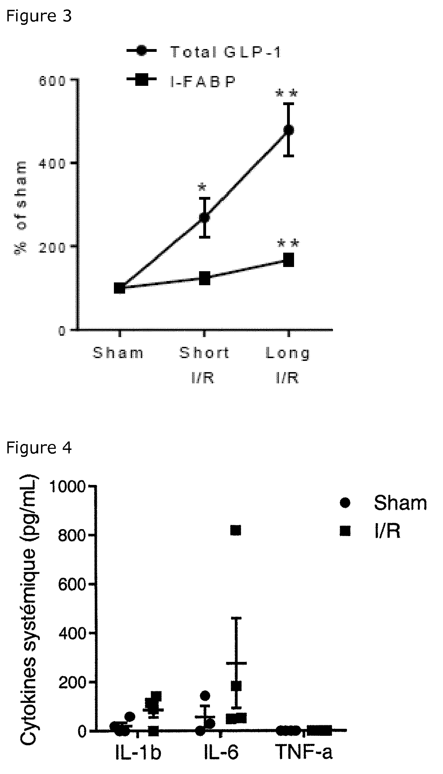

It is revealed that after mesenteric ischemia-reperfusion, GLP-1 secretion precedes I-FABP secretion (Figure 3) and markers of inflammation, such as IL-1b, IL-6, TNF-a (Figure 4). In contrast to I-FABP, a short I/R treatment was sufficient to induce a significant plasma GLP-1. Moreover, after mesenteric ischemia-reperfusion, GLP-1 secretion is quantitatively more important than that of I-FABP (Figure 3).

These results show that, compared with I-FABP, GLP-1 is more sensitive and can be used as biomarker for diagnosing gut barrier injury at more early stage.

## Claims

1. An *in vitro* diagnostic method for diagnosing at an early stage an intestinal ischemia in a patient suspected of suffering from intestinal ischemia, which comprises:
(i) determining in a biological sample from said patient the circulating glucagon-like peptide 1 (GLP-1) level,
(ii) comparing said level with the circulating glucagon-like peptide 1 level in a reference sample,
wherein the increase of said level in the patient is indicative of said patient suffering from intestinal ischemia.

2. The method according to claim 1, wherein the intestinal ischemia is mesenteric ischemia-reperfusion, especially acute mesenteric ischemia, chronic mesenteric ischemia, or ischemic colitis, or a disease or trouble linked to gut barrier.

3. The method according to claim 1 or 2, wherein the biological sample is a blood plasma sample.

4. The method according to any one of claims 1 to 3, wherein the level of circulating glucagon-like peptide 1 is determined by the means of an immunoassay.

5. The method according to claim 4, wherein said immunoassay is ELISA.

6. The method according to any one of claims 1 to 5, wherein the reference sample is a biological sample from a healthy subject.

7. Use of a reagent capable of detecting the level of circulating glucagon-like peptide 1 for diagnosing *in vitro* at an early stage an intestinal ischemia in a patient suspected of suffering from intestinal ischemia.

8. The use according to claim 7, wherein the reagent is an antibody directed against glucagon-like peptide 1.

9. Use of a kit of ELISA containing an antibody directed against glucagon-like peptide 1 for diagnosing *in vitro* at an early stage an intestinal ischemia in a patient suspected of suffering from intestinal ischemia.
